# EUROPEAN PATENT SPECIFICATION

(11) **EP 0 610 286 B1**
(45) Date of publication and mention of the grant of the patent: **15.03.2000**
(21) Application number: 92921866.7
(22) Date of filing: 29.09.1992
(51) Int. Cl.: C12N 15/62, C12N 15/31, C12N 15/13, C12N 1/21, C07K 16/00, C07K 16/28, A61K 39/104, A61K 39/395

(54) **RECOMBINANT IMMUNOTOXINS**
REKOMBINANTE IMMUNTOXINE
IMMUNOTOXINES RECOMBINEES

(30) Priority: 30.09.1991 US 767331
(43) Date of publication of application: 17.08.1994
(73) Proprietor: THE UNITED STATES OF AMERICA, as represented by the Secretary of the Department of Health and Human Services, Bethesda, Maryland 20892 (US)
(72) Inventor: PASTAN, Ira, Potomac, MD 20854 (US); WILLINGHAM, Mark, Bethesda, MD 20814 (US); FITZGERALD, David, Silver Spring, MD 20902 (US); BRINKMANN, Uli, Bethesda, MD 20814 (US); PAI, Lee, Silver Spring, MD 20906 (US)
(74) Representative: Kirkham, Nicholas Andrew
(86) International application number: US9208257
(87) International publication number: WO9307286

(56) References cited:
- WO-A-90/12592
- PROC. NATL. ACAD. SCI. U. S. A., 88(8), 3358-62 CODEN: PNASA6;ISSN: 0027-8424, 15 April 1991 PAI, LEE H. ET AL 'Antitumor activities of immunotoxins made of monoclonal antibody B3 and various forms of Pseudomonas exotoxin'
- THE JOURNAL OF BIOLOGICAL CHEMISTRY, vol. 266, no. 26, 15 September 1991 pages 17376-17381, SARASWATHY SEETHARAM ET AL. 'Increased cytotoxic activity of Pseudomonas exotoxin and two chimeric toxins ending in KDEL'
- PROCEEDINGS OF THE NATIONAL ACADEMY OF SCIENCES OF USA, vol. 87, no. 23, December 1990 WASHINGTON US, pages 9491-9494, VIJAY K. CHAUDHARY ET AL. 'A recombinant single-chain immunotoxin composed of anti-Tac variable regions and a truncated diphteria toxin'
- PROCEEDINGS OF THE 82ND ANNUAL MEETING OF THE AMERICAN ASSOCIATION FOR CANCER RESEARCH, HOUSTON, TEXAS, USA, MAY 15-18, 1991. PROC AM ASSOC CANCER RES ANNU MEET 32 (0). 1991. 261. CODEN: PAMREA, PAI L H ET AL 'ANTI-TUMOR ACTIVITIES OF IMMUNOTOXINS MADE OF MONOCLONAL ANTIBODY B3 AND DIFFERENT FORMS OF PSEUDOMONAS EXOTOXIN.'
- PROC. NATL. ACAD. SCI. U. S. A., 88(19), 8616-20 CODEN: PNASA6;ISSN: 0027-8424, 1 October 1991 BRINKMANN, ULRICH ET AL 'B3 (Fv)-PE38KDEL, a single-chain immunotoxin that causes complete regression of a human carcinoma in mice'
- Cancer Research, Volume 51, issued 15 July 1991, IRA PASTAN et al., "Characterization of Monoclonal Antibodies B1 and B3 that React with Mucinous Adenocarcinomas", pages 3781-3787, see entire document.
- Journal of Biological Chemistry, Volume 263, No. 19, issued 05 July 1988, TOSHIHIKO KONDO et al., "Activity of Immunotoxins constructed with Modified Pseudomonas Exotoxin A lacking the Cell Recognition Domain", pages 9470-9475, see entire document.
- Journal of Biological Chemistry, Volume 265, No. 25, issued 05 September 1990, JANENDRA K. BATRA et al., "Anti-Tac(Fv)-PE40: A Single Chain Antibody Pseudomonas Fusion Protein Directed at Interleukin 2 Receptor Bearing Cells", pages 15198-15202, see entire document.
- Proc. Natl. Acad. Sci. USA, Volume 87, issued 01 February 1990, VIJAY K. CHAUDHARY et al., "A rapid method of cloning functional variable-region antibody genes in Escherichia coli as single-chain immunotoxins", pages 1066-1070, see entire document.
- Nature, Volume 339, No. 6223, issued 01 June 1989, VIJAY K. CHAUDHARY et al., "A recombinant immunotoxin consisting of two antibody variable domains fused to Pseudomonas exotoxin", pages 394-397, see entire document.
- Harlow and Lane, "Antibodies: A Laboratory Manual", Cold Spring Harbor Laboratory 1988, page 29

## Description

The subject invention relates to recombinant immunotoxins, and to uses thereof. In particular, the invention relates to two immunotoxins, referred to as B3(Fv)-PE40 and B3(Fv)-PE38KDEL, which may be used in the treatment of mammalian cancer.

### BACKGROUND INFORMATION

The monoclonal antibody B3 (MAb B3) is a recently isolated murine antibody which is directed against a carbohydrate antigen in the LE family (Pai et al., Proc. Natl. Acad. Sci. USA 88:3358-62 (1991)). This antigen is found on the surface of many mucinous carcinomas of the colon, stomach, ovaries, breast, lung as well as some epidermal carcinomas. Because it reacts with only a limited number of normal tissues, MAb B3 is an ideal candidate for the treatment and diagnosis of cancer. In order to create a cytotoxic agent, MAb B3 has been previously chemically coupled to two different forms of Pseudomonas exotoxin (PE) (U.S. patent 4,545,985). One of these is the full length toxin (PE) and the other a truncated derivative (PE40) (Kondo at al., J. Biol. Chem. 263:9470-75 (1988) & Pai at al., supra). Both of these immunotoxins have been shown to be selectively cytotoxic to tumor cells that contain the B3 antigen on their surface, and these immunotoxins have also been shown to cause complete tumor regression in mice bearing human tumor xenografts (Pai et al., Proc. Natl. Acad. Sci. USA 88:3358-62 (1991)). Although these first generation immunotoxins have properties that indicate they should be developed further as drugs for the treatment of cancer, immunotoxins made by chemical conjugation methods have several undesirable properties. For example, the chemical modifications can change the antibody and affect its binding to the antigen. Furthermore, the purified immunotoxins are a heterogeneous mixture of antibody-toxin molecules connected to each other via different positions on the antibody and the toxin. Thus, PE can be coupled either to the light- or heavy-chain of the antibody and to different positions on each of these chains.

The second generation of immunotoxins are made as recombinant antibody Fv-fusion proteins in bacteria. It has been shown that single chain antigen binding proteins (scAB's, scFv's) made from the Fv portions of the heavy and light chain of antibodies held together by a polypeptide linker can have the same binding properties as their full length two chain counterparts (Bird et al., Science 242:423-26 (1988) and Huston et al., Proc. Natl. Acad. Sci. USA 85:5879-83 (1988)). Furthermore, fusion proteins composed of scAB's linked to toxins retain the binding capacity of the scAB as well as the activity of the toxin (Chaudhary et al., Nature 339:394-97 (1989); Batra et al., J. Biol. Chem. 265:15198-202 (1990); Batra et al., Proc. Natl. Acad. Sci. USA 86:8545-49 (1989); Chaudhary et al., Proc. Natl. Acad. Sci. USA 87:1066-70 (1990)).

Potent single chain immunotoxins have been made previously by fusing the Fv domains of antibodies directed at the interleukin 2 receptor (Chaudhary et al., Nature 339:394-97 (1989) & Batra et al., J. Biol. Chem. 265:15198-15202 (1990)) or at the transferrin receptor (Batra et al., Proc. Natl. Acad. Sci. USA 86:8545-49 (1989)) to truncated forms of PE or diphtheria toxin (Chaudhary et al., Proc. Natl. Acad. Sci. USA 87:9491-94 (1990)). Receptors often make good immunotoxin targets because they are cell surface proteins that can be rapidly internalized, and toxins must be internalized in order to kill cells.

WO90/12592 describes a recombinant immunotoxin comprising an antibody-PE40 recombinant single chain fusion protein. This immunotoxin has been shown to have a cytotoxic effect on cells possessing particular antigens or receptors.

The immunotoxins described herein are selectively cytotoxic to cultured mammalian tumor cells bearing the B3 antigen and also cause regression of mammalian tumors in vivo. These immunotoxins are described in detail below.

The present invention relates to immunotoxins which may be used for the treatment of mammalian cancer.

In particular, the present invention relates to the recombinant DNA molecule of claim 1, the host cell of claim 3, the single chain immunotoxin of claim 5, the medicament of claim 8, the method of producing an immunotoxin of claim 9 and the medical uses of claims 10 and 11.

Again, the vector utilized in creating the recombinant molecule may be, for example, pULI1, pULI3, a derivative of either of these plasmids, or any other vector which allows for the expression of recombinant proteins in microorganisms. The bacterial toxin referred to above is Pseudomonas exotoxin.

A derivative of a plasmid or gene would include changes in the DNA sequence of the plasmid or gene, respectively, that do not eliminate either the antigen binding of the Fv fragment or the translocating and enzymatic activity of the toxin.

The present invention also includes a host cell stably transformed with the recombinant DNA molecule of in a manner allowing expression of the immunotoxin encoded by the recombinant DNA molecule. The host cell may be a procaryotic cell such as, for example, an Escherichia coli cell.

Furthermore, the present invention also relates to a recombinantly produced immunotoxin consisting of a toxin protein or a portion thereof and the Fv region of the light and heavy chains of a monoclonal antibody. Once again, the toxin utilized is Pseudomonas exotoxin. The monoclonal antibody utilized is MAb B3.

The present invention also encompasses a composition comprising the immunotoxin described directly above and a pharmaceutically acceptable carrier.

The invention also includes a method of treating cancer in a patient comprising administering, to the patient, an amount of the composition described-above, sufficient to effect the treatment.

The immunotoxins of the present invention are created by:
(i) cloning a DNA sequence which encodes the Fv region of both the heavy and light chain of a monoclonal antibody into a vector, the vector comprising a gene which encodes an altered form of a bacterial toxin or a portion of the toxin; and
(ii) transforming a host cell with the resulting vector of step (i), thereby allowing for expression of the immunotoxin.

Again, the altered form of the toxin retains translocating and enzymatic activity.

The monoclonal antibody which may be utilized in the production of the immunotoxin is MAbB3. This monoclonal antibody preferentially reacts with human tumors.

The immunotoxin which can be produced is selected from the group consisting of B3(Fv)-PE40 and B3(Fv)-PE38KDEL. For example, B3(Fv)-PE38KDEL can be created as a derivative of B3(Fv)-PE40 by deleting sequences in the Ib domain of B3(Fv)-PE40 and by changing the carboxy terminus of the molecule to increase the cytoxicity thereof.

The vector used in the method of forming the immunotoxin may be, for example, pULI1, pULI3, a derivative of either of these plasmids, or any other vector that allows for the expression of recombinant proteins in microorganisms. The bacterial toxin, referred to above, is Pseudomonas exotoxin (PE).

It should be noted that, prior to the present invention, no single chain immunotoxin had been shown to have an antitumor effect in a mammal. Furthermore, the anti-tumor effect of the present immunotoxins is quite remarkable as complete tumor regression may be exhibited in only a few days with only a small amount of the relevant immunotoxin being required. Such a result requires a molecule which can enter the tumor, effectively bind to the tumor cells, and subsequently kill these cells. Thus, the molecule must penetrate into the interior of the tumor cells. It is thought that the immunotoxins of the present invention possess such an ability or such significant anti-tumor effects would not otherwise be observed.

### BRIEF DESCRIPTION OF THE DRAWINGS

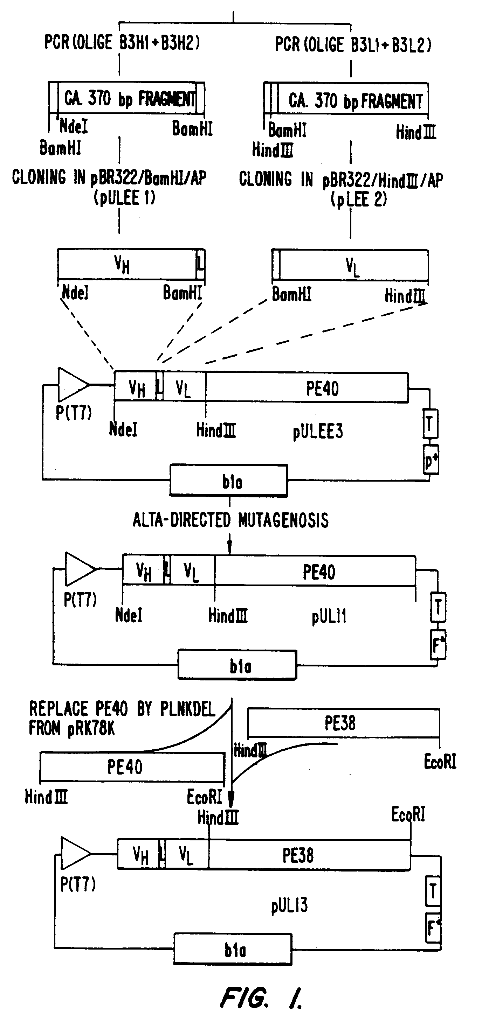

FIGURE 1 represents the strategy for the cloning of the heavy and light chain Fv genes of MAb B3 and the construction of expression vectors (e.g., plasmids) for the expression of B3(Fv) immunotoxins. The cloning strategy is a variation of that previously described (Chaudhary et al., Proc. Natl. Acad. Sci. USA 87:1066-70 (1990)). The plasmid pVC38H, which is used as a vector for construction of immunotoxins from heavy and light chain Fv regions, contains an NdeI and a HindIII recognition sequence preceding the PE40 gene (Chaudhary et al., supra (1990)). The sequences of the PCR primers are shown in Example I. (*) indicates a PCR-generated mutation and was repaired by site directed mutagenesis; (L) indicates the region encoding the (Gly,Ser), linker which serves to join heavy and light chains of the immunotoxin.)

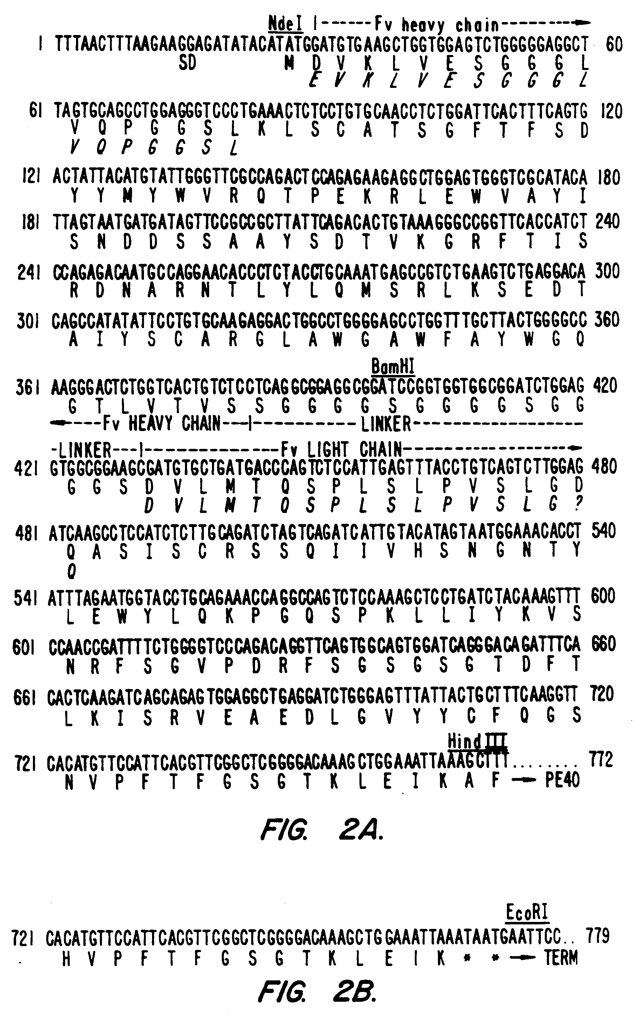

FIGURE 2 shows the nucleotide sequences encoding the heavy and light chain Fv region of MAb B3. (a) - the heavy chain Fv coding region extends from position 30 to 383, the light chain Fv gene from position 433 to 767 and the linker from 384 to 432. The deduced amino acid sequence is shown in plain letters; below in italic letters is the protein sequence determined by Edman sequencing of the antibody. The first amino acid encoded by the cloned heavy chain Fv gene is Asp instead of Glu due to the oligonucleotide primer used, at position 456-465 is the region where the PCR cloning artifact was repaired. This sequence encodes the same amino acids as the original B3 light chain gene but uses other codons. Homology comparisons to the known nucleotide sequence of PAC1 Ig kappa chain (Taub et al., J. Biol. Chem. 264:259-65 (1989)) which is most homologous to the B3 light chain indicates that the original sequence was most probably CTCTCCCTG instead of TTGAGTTTA. Thus the natural B3 light chain gene has a sequence repetition 5'(CCAGTCT[CC)ACTCTCC]3' between positions 445 and 465 which is responsible for the incorrect primer annealing in PCR. (b) - sequence at the 3'-end of the light chain for expression of the single chain B3(Fv) alone. (SD) - Shine Dalgarno consensus sequence; (*) - translation stop signal. (Term) - transcription terminator.

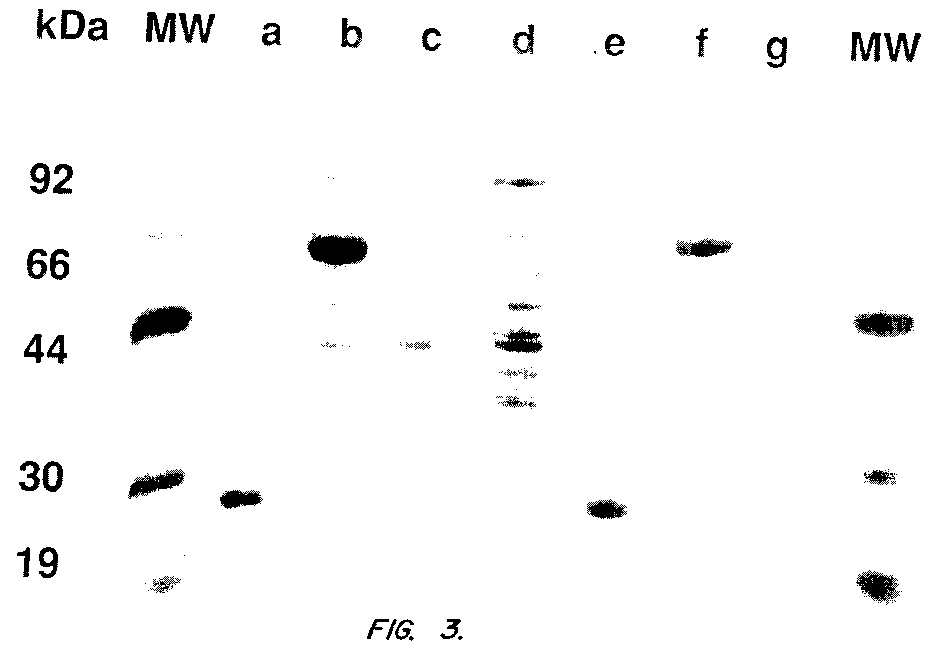

FIGURE 3 shows the recombinant B3(Fv) and B3(FV) immunotoxins from bacterial inclusion bodies. SDS-PAGE (24) (12.5%) showing (a) total cell protein of induced bacteria producing single chain B3(Fv); (b) total protein of cells producing B3(Fv)-PE40; (c,d) supernatant of sonicated cells producing (c) B3(Fv) or (d) B3(Fv)-PE40; (e) inclusion bodies containing B3(Fv); (f) inclusion bodies containing B3(Fv)-PE40 ; (g) purified B3(Fv)-PE40 protein after gel filtration. MW: molecular weight standard.

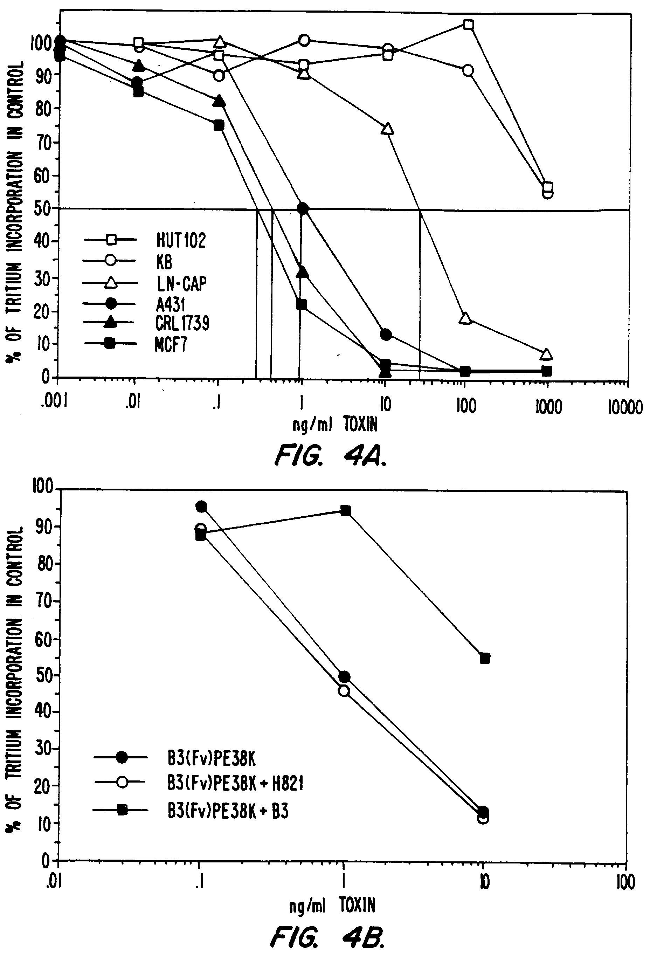

FIGURE 4(a) represents the toxicity of B3(Fv)-PE38KDEL on different cell lines. Cytotoxicity assays were performed as described in Example IV. (b): Inhibition of the cytotoxicity of B3(Fv)-PE38KDEL by MAb B3. Competition by MAb B3 was performed on A431 cells as described in Example IV.

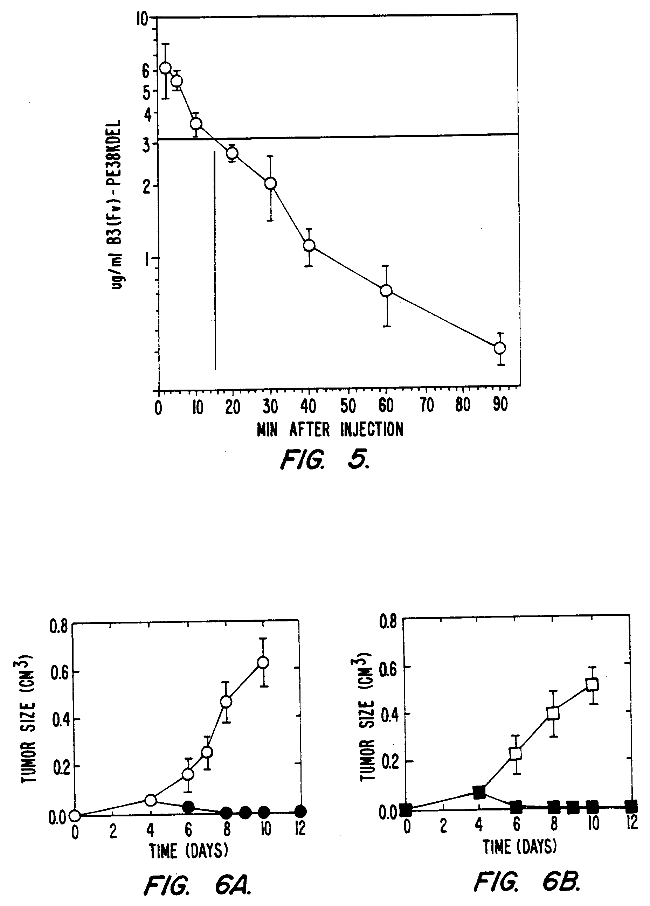

FIGURE 5 shows blood levels of B3(Fv)-PE38KDEL in mice. Balb/c mice were injected i.v. with 10 µg of B3(Fv)-PE38KDEL and immunotoxin levels measured at different time periods. Bars indicate the standard deviation.

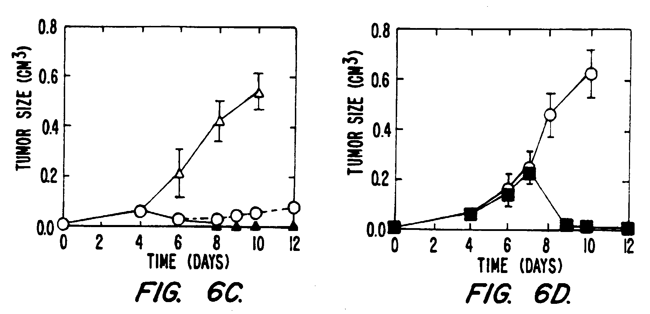

FIGURE 6 represents the effect of B3(Fv)-PE38KDEL on the growth of A431 tumors in nude mice. Mice were injected with 3x10⁶ A431 cells on day 0 and treated beginning on day 4 with i.v. injections every 12 hrs x 6. A: (○) untreated; (●) 10 µg B3(Fv)-PE38KDEL; B: (□) 2.5 µg B3; (■) 5 µg B3(Fv)-PE38KDEL; C: (△) 2.5 µg anti-Tac (Fv)-PE38KDEL; (▲) 2.5 µg B3(Fv)-PE38KDEL; (--○--) 0.5 µg B3(Fv)-PE38KDEL; D: treatment began on day 7 with i.v. injections every 12 hrs x 8. (○) untreated, (■) 5 µg B3(Fv)-PE38KDEL. Bars = standard deviation.

### DETAILED DESCRIPTION OF THE INVENTION

In order to prepare the immunotoxins of the present invention, initially sequences encoding the heavy and light chain Fv domains of the murine monoclonal antibody B3 are cloned. As mentioned above, this MAb recognizes an antigen present on many carcinoma cells, and may be useful for the treatment of various types of cancers (Pai et al., Proc. Natl. Acad. Sci. USA 88:23358-62 (1991). The heavy and light chain regions of MAb B3 are then connected by a flexible linker (Gly,Ser), which starts at the carboxyl end of the heavy chain Fv domain and ends at the amino terminus of the light chain Fv domain. The resulting gene encodes the B3(Fv) domain in the form of a single chain antigen binding protein. This B3(Fv) gene is then fused to sequences encoding two different truncated forms of the PE molecule to obtain single chain B3(Fv) immunotoxins. These recombinant immunotoxins can kill carcinoma cells containing the B3 antigen without affecting control cells. The cytotoxicity of the recombinant B3(Fv)-PE40 is similar to a chemical conjugate of B3 and PE40 (B3-LysPE40) (Pai et al., supra (1991)), but B3(Fv)-PE38KDEL is five-fold more active (see Table 1). Thus, less material needs to be administered to patients which results in a better anti-tumor effect, minimal side effects as well as a diminished production of neutralizing antibodies to the recombinant toxin. Moreover, since B3(Fv)-PE38KDEL is much smaller in size than B3-LysPE40, it will penetrate tumors far more effectively.

Furthermore, B3(Fv)-PE38KDEL causes complete regression of A431 tumors grown in immunodeficient mice. This makes the B3(Fv) derived single chain immunotoxins a promising alternative to B3 chemical conjugates and a possible second generation immunotoxin for the treatment of solid tumors. For example, the immunotoxins could be administered either intravenously for cancers which have spread or could also be administered locally (i.e., into the bladder for use in the treatment of bladder cancer or into the peritoneal cavity for use in the treatment of ovarian cancer). The immunotoxins may be given for 7-10 days, for example. The treatment could then be repeated as often as necessary.

The authenticity of the cloned DNA fragments can be proven by comparing the amino terminal protein sequences of the B3 heavy and light chains with the amino acid sequences deduced from the reading frames of the cloned genes (FIG. 2). The sequences of the cloned Fv coding regions are shown in Figure 2.

The analysis of the cytotoxicity of B3-immunotoxins showed the same sensitivity-pattern of different cell lines towards the recombinant immunotoxins as towards the chemical conjugates. All these immunotoxins were very cytotoxic to carcinoma cells that express the B3 antigen on their surface including MCF7 (breast), A431 (epidermoid), CRL1739 (gastric) and LNCaP (prostate). Thus, all of these cell lines, as well as many others, react with the B3 antibody. (See Table 1 below.)

**Table 1**

| Activities of B3 immunotoxins on different cell lines Cytotoxicity (ID₅₀) in ng/ml (pM) | | | | | |
|---|---|---|---|---|---|
| Cell Line | Cancer Type | B3 antigen | B3(Fv)·PE40 | B3(Fv)·PE38KDEL | B3·LysPE40 |
| MCF7 | breast | ++ | 3 (50) | 0.2 (3.2) | 3 (16) |
| CRL1739 | gastric | ++ | 3 (50) | 0.3 (5) | 3 (16) |
| A431 | epidermoid vulva | + | 3 (50) | 0.8 (13) | 8 (42) |
| LMCaP | prostate | + | 40 (1330) | 20 (325) | 85 (460) |
| KB3-1 | epidermoid cervix | - | >1000 | >1000 | >1000 |
| HUT102 | adult T cell leukemia | - | >1000 | >1000 | >1000 |

Also, the recombinant single chain B3-Fv imnunotoxins did not affect B3 antigen-negative control cells. The cytotoxicity of the recombinant B3(Fv)-PE40 (ID₅₀ = 50 pM; 3.0 ng/ml) was similar to the chemically linked B3-immunoconjugate (ID₅₀ = 42 pM; 8 ng/ml), whereas B3(Fv)-PE38KDEL was much more active than the chemical conjugate (ID₅₀ 13 pM; 0.8 ng/ml). This is despite the fact that the single chain immunotoxins possess only one antigen binding site per molecule and the chemical conjugate has two (see Table 2 below).

**Table 2**

| Structure and Activity of B3 Immunotoxins on A431 Cells | | | | |
|---|---|---|---|---|
| Immunotoxin | Toxin Part | C-Term | Binding | ID₅₀ |
| B3 chemical conjugate | PE40 | REDLK | bivalent | 8.0 ng/ml (42 pMol) |
| B3(Fv) fusion protein | PE40 | REDLK | monovalent | 3.0 ng/ml (50 pM) |
| B3(Fv) fusion protein | PE38 | KDEL | monovalent | 0.8 ng/ml (13 pM) |

B3(Fv)-PE38KDEL has two features that distinguish it from B3(Fv)-PE40. One is that a portion of domain Ib encompassing amino acids 365-380 is deleted. This removes the disulfide bond formed between cysteine residues at positions 372 and 379, which might form disulfide bonds with other cysteines during the renaturation process and thereby result in the creation of inactive chimeric toxins. The second feature is that the carboxyl terminus of the toxin is changed from the original sequence REDLK to KDEL. When the disulfide bond was removed in other molecules, the increase in activity was small. For example, TGFα-PE38 is only 50% more active than TGFα-PE40 (see Siegall et al., J. of Biol. Chem. 264:14256-61 (1989)). IL6PE38 is no more active than IL6-PE40. Changing REDLK to KDEL usually only produces a two to three fold increase in activity of chimeric toxins.

B3(Fv)-PE38KDEL was tested for its antitumor effect in nude mice bearing A431 tumors. Complete regression of tumors was observed when mice received 2.5 µg, 5 µg or 10 µg of the chimeric toxin twice daily for three days, despite the fact that B3(Fv)-PE38KDEL has a short lifetime (15-20 min) in the circulation. B3(Fv)-PE38KDEL also produced complete regression of tumors about 1 cm in diameter. Previously, it was found that even the administration of 75 µg per day for 5 days of a chemical conjugate composed of B3 and PE40 (see Table 2) only produced partial regression of large tumors despite the fact that the chemical conjugate has a much longer lifetime in the blood (4 hrs.). The recombinant molecule probably has a higher antitumor activity in the mouse model because of its small size which allows better access to tumor cells.

The present invention can be illustrated by the use of the following non-limiting examples:

### Example I

### Cloning of DNA Fragments Encoding the Heavy and Light Fv Region of MAb B3

Cloning experiments and propagation of plasmids were carried out generally in E. coli HB101 (Boyer at al., J. Molec. Biol. 41:459-72 (1969)). DNA fragments encoding the Fv portions of heavy and light chain of MAb B3 were obtained by (PCR-) amplification of single stranded DNA which was synthesized by random primed reverse transcription of mRNA from a MAb B3 producing hybridoma cell line. Polymerase chain reaction (Saiki et al., Science 239:487-91 (1988)) was performed using the Perkin Elmer GeneAmp kit and an Perkin Elmer/Cetus thermocycler, under conditions as described (Chaudhary at al., Proc. Natl. Acad. Sci. USA 87:1066-70 (1990)). For amplification of the heavy chain Fv coding region, the present inventors chose the primer pair B3-H1 (5'TAACTAGGATCCGTCCATATG GATGTGAAGCTGGTGGAGTCTGG3') and B3-H2 (5'TGGATAGACTG ATGGGGATCCGCCTCCGCCTGAGGAGAC3') and for the light chain B3-L1(5'GTCTCCAAGCTTGGGGATCCGGTGGTGGCGGATCTGG AGGTGGCGGAAGCGATGTGCTGACCCAGTCTCC3') and B3-L2(5'AGTTGGTGCAGCATCAAAAGCTTT[G/T]A[G/T][T/C]TCCAGCT T[T/G]GT]G/C]CC3'). These oligonucleotides have at their 3' end constant sequences that occur at the beginning and end of mouse Fv DNA. At their 5' end are restriction endonuclease recognition sites (NdeI, BamHI, HindIII) for cloning of the PCR products as shown in FIG. 1. The products of the amplifications of heavy- and light chain Fv DNA fragments were identified by agarose gel electrophoresis to be DNA fragments between 350 and 400 bp. They were purified from gels, cut with BamHI or HindIII (FIG. 1) and after purification on a second gel, ligated with HindIII- or BamHI-linearized and dephosphorylated pBR322 vector (Bolivar et al., Gene 2:95-113 (1977)). The nucleotide sequence of the light- and heavy chain Fv coding region of MAb B3 was determined from double stranded plasmid DNA using sequencing primers (New England Biolabs) adjacent to the BamHI or HindIII site of pBR322 and a T7 polymerase sequencing reagent kit (United States Biochemicals).

### Example II

### Construction of Plasmids for Expression of B3(Fv) and B3(Fv)-Immunotoxins

The expression plasmid pVC38H contains the gene from the immunotoxin TGFα-PE40 under control of the T7 promoter (Chaudhary et al., Proc. Natl. Acad. Sci. USA 87:1066-70 (1990)), the Tφ transcription terminator at the 3'-end of the PE40 coding region and the single strand replication origin, F+, to generate single stranded phage DNA by cotransfection with (M13) helper phages, if desired, to create derivatives of the plasmid by site directed mutagenesis. The TGFα coding region in pVC38H has an NdeI recognition site at the 5'-end and a HindIII site at the point of connection to the DNA encoding PE40. To create a plasmid for expression of the immunotoxin B3(Fv)-PE40 (pULEE3), the TGFα gene was removed and replaced by the B3(Fv) gene in a 3-fragment ligation, using an NdeI/BamHI fragment of the heavy chain coding region and the BamHI/HindIII fragment encoding the light chain Fv (FIGURE 1). Because sequence analysis showed a mutation (deletion and frameshift) at the 5' end of the light chain Fv gene due to a sequence repetition in the PCR primer annealing region, site-directed mutagenesis was performed (Kunkel, T.A., Proc. Natl. Acad. Sci. USA 82:488-92 (1985)), using uridine incorporated single stranded phagemid DNA (pULEE3) as the mutagenesis template. In the resulting plasmid (pULI1), the correct amino end of the B3 light chain established by partial protein sequencing of MAb B3, is reconstructed.

To make another B3(Fv) immunotoxin, B3(Fv)-PE38DKEL, the PE40 coding region was removed from pULI1 from the HindIII site to an EcoRI site positioned just beyond the PE40 gene, and replaced by a HindIII/EcoRI fragment from pRK79K encoding the PE variant PE38KDEL which lacks domain Ia (amino acids 1-252) and part of domain Ib (amino acids 365-380), and also contains an altered carboxyl terminal sequence KDEL (Chaudhary et al., Proc. Natl. Acad. Sci. 87:308-12 (1990)). The expression plasmid pULI4 for production of B3(Fv) was constructed by removal of the light chain and PE40 coding region from pULI1 from BamHI to EcoRI which was replaced by a PCR fragment obtained by amplification of the light chain Fv coding sequence with the primer-pair B3-L3 + B3-L4. The primer B3-L3 (5'TTGGGGATCCGGTGGTGGCGGATCTGGA3') is similar to B3-L1, used for cloning of light chain Fv from cDNA and B3-L4 ('AGCGGGAATTCATTATTTAATTTCCAGCTTTGTCCCCGAC3') is in the 3' part for priming the PCR identical to B3-L2, but at the 5' end the HindIII site for fusion to PE-sequences is replaced by translation stop codons followed by an EcoRI recognition sequence.

### Example III

### Expression and Purification of Recombinant B3 (Fv) - Immunotoxins

Plasmids were transformed in the expression-host E. coli BL21 (λDE3) (Studier et al., J. Mol. Biol. 189:113-30 (1986)). The bacteria were grown in superbroth containing 2% glucose, 0.05 % MgSO₄ and 100 µg/ml ampicillin, induced in the log phase at OD₆₀₀ of 3.0 with 1 mM IPTG and harvested 90 min later. About 30% of the total protein of the induced cultures was the recombinant expression product which was deposited in inclusion bodies. The purified inclusion bodies contained almost pure recombinant protein, which had the expected size of about 67 kDa for a single chain immunotoxin (FIGURE 3). The recombinant immunotoxin molecules were solubilized, refolded, purified, and the protein was analyzed as previously described (Chaudhary at al., Nature 339:394-97 (1989) & Batra et al., J. Biol. Chem. 265:15198-202 (1990)). Protein concentrations were determined by Bradford assay (Bradford, M.M., Anal. Biochem. 72:848-54 (1976)). The purity of the molecules is shown in FIGURE 3.

### Example IV

### Cytotoxic Activity of Chemically Linked and Recombinant Immunotoxins

Assays measuring inhibition of protein synthesis were previously described (Chaudhary at al., Nature 339:394-97 (1989) & Batra et al., J. Biol. Chem. 265:15198-202 (1990)). All assays were performed in 96 well plates each well containing 1.6 x 10⁴ cells in 200 µl medium. For competition assays designed to prove the specificity of the recombinant immunotoxins, the medium was changed and 50 µg/well of antibody was added 15 min prior to the addition of the immunotoxin.

As shown in Figure 4 and in Table 1, the recombinant single chain immunotoxins inhibited protein synthesis in cells expressing the B3 antigen but not in non-expressing cells, similarly to the previously described results with chemical conjugate of B3 with a truncated form of PE (Pai et al., Proc. Natl. Acad. Sci. USA 88:3358-62 (1991)). The relative potencies of the chemical conjugate and the single chain immunotoxins were about the same on the four antigen positive cell lines MCF-7, CRL 1739, A431 and LNCaP. The most active agent was B3(Fv)-PE38KDEL. To analyze whether the cytotoxicity of B3(Fv)-immunotoxins was specific, competition experiments were carried out with an excess of MAb B3. The data in FIGURE 4b shows that the intoxication of A431 carcinoma cells by B3(Fv)-PE38KDEL is due to the specific binding to the B3 antigen, since its cytotoxicity was blocked by excess MAb B3 but not by MAb HB21 which recognizes the transferrin receptor on these cells (Haynes et al., J. Immunol. 127:347-51 (1981)). A large excess of MAb B3 is necessary for reversal of cytotoxicity, probably because there is a large amount of the B3 antigen on the surface of A431 cells (Pai et al., Proc. Natl. Acad. Sci. USA 88:23358-62 (1991)).

### Example V

### Assay of Blood Levels of B3(Fv)-PE38KDEL in Mice

Six week old (19-20 gm) female Balb/c mice were injected with 10 µg of B3(Fv)- PE38KDEL in the tail vein. Blood was drawn at various time intervals and the level of the immunotoxin measured by incubating serum with A431 cells and measuring inhibition of protein synthesis. A standard curve was made with pure B3(Fv)-PE38KDEL and the blood level of immunotoxin (which is shown in Figure 5) calculated using this curve.

### Example VI

### Anti-Tumor Activity of B3(Fv)-PE38KDEL in Nude Mice Bearing a Human Epidermoid Carcinoma

A431 cells (3 x 10⁶) were injected subcutaneously on day 0 into female nude mice (4-6 weeks old, 18-20 gm). Mice with 5 mm by 5 mm tumors, that usually developed by day 4, were treated with B3(Fv)-PE38KDEL or as a control with MAbB3 or anti-Tac(Fv)-PE38KDEL (Chaudhary et al., Nature 339:394-97 (1989)). Because the lifetime of B3(Fv)-PE38KDEL in the circulation of the mice was observed to be only 15-20 min (FIGURE 5), six injections were given at 12 hour intervals into the tail vein, starting 4 days after tumor implantation. Each treatment group consisted of five animals. The volume of the tumor was calculated by [tumor volume in cm³=length x width² x 0.4].

As shown in FIGURE 6, injection of either 2.5, 5 or 10 µg twice daily produced complete tumor regression. Partial regression was observed when only 0.5µg was injected. No toxicity was observed at these doses. In addition, when mice with large tumors about 1 cm in diameter were treated with 5µg twice a day for 4 days, complete regression of these large tumors containing about 5 x 10⁸ cells rapidly occurred (FIGURE 6D). Regression of MCF-7 tumors (breast carcinoma) also was observed with 5µg twice daily of B3(Fv)-PE38KDEL. In control experiments, mice were treated with either MAb B3 or anti-Tac(Fv)-PE38KDEL, which is cytotoxic to cells with IL2 receptors but not for A431 cells (Chaudhary et al., supra (1989)) and no antitumor effect was observed.

## Claims

1. A recombinant DNA molecule comprising:
i. a DNA sequence which encodes the Fv region of both the light and heavy chains of monoclonal antibody B3; and
ii. an altered form of a bacterial toxin selected from the group consisting of PE40 and PE38KDEL as described in Example II, wherein when expressed said Fv region and said toxin are recombinantly fused to form a single chain immunotoxin that has the binding specificity of monoclonal antibody B3.

2. The recombinant DNA molecule of claim 1, wherein said molecule encodes an immunotoxin selected from the group consisting of the B3(Fv)-PE40 and B3(Fv)-PE38KDEL as immunotoxins described in Example II.

3. A host cell transformed with the recombinant DNA molecule of claim 1 or 2 in a manner allowing expression of an immunotoxin encoded by said recombinant DNA molecule.

4. The host cell according to Claim 3, wherein the cell is a procaryotic cell optionally an *Escherichia coli* cell.

5. A recombinantly produced single chain immunotoxin comprising:
i. the Fv region of both the light and heavy chains of an antibody; and
ii. an altered form of a bacterial toxin selected from the groups consisting of PE40 and PE38KDEL as described in Example II, wherein said Fv region and said toxin are recombinantly fused to form a single chain immunotoxin that has the binding specificity of monoclonal antibody B3.

6. The immunotoxin of claim 5, wherein the monoclonal antibody is B3.

7. The immunotoxin of claim 5 or 6, wherein said immunotoxin is selected from the group consisting of B3(Fv)-PE40 and B3(Fv)-PE38KDEL as defined in Claim 2.

8. A medicament comprising a recombinantly produced single chain antigen binding protein fused to a toxin or a portion thereof, in a concentration sufficient to inhibit tumor cell growth, together with a pharmaceutically acceptable carrier, wherein said fused protein comprises:
i. a single chain Fv region of antibody B3, said Fv region comprising the V_{H} and V_{L} regions of said antibody; and
ii. a toxin molecule selected from the group consisting of PE40 and PE38KDEL defined in Claim 1.

9. A method of producing an immunotoxin comprising the steps of:
i. cloning a DNA sequence which encodes the Fv region of both the heavy and light chain of monoclonal antibody B3 into a vector, said vector comprising a gene which encodes an altered form of a toxin selected from the group consisting of PE40 and PE38KDEL defined in Claim 1, said altered form retaining translocating and enzymatic activity; and
ii. transforming a host cell with the resulting vector of step (i), thereby allowing for expression of said immunotoxin.

10. The recombinant DNA molecule of claim 1 or 2, the host cell of Claim 3, the single chain immunotoxin of any one of claims 5 to 7, or the medicament of claim 8, for use in therapy, preferably treatment of cancer in a mammal.

11. Use of the recombinant DNA molecule of claim 1 or 2, the host cell of claim 3, the single chain immunotoxin of any one of claims 5 to 7 or the medicament of claim 8, for the manufacture of a medicament for treating cancer.

## Patentansprüche

1. Rekombiniertes DNA-Molekül aus:
i. einer DNA-Sequenz, welche die Fv-Region sowohl der leichten als auch der schweren Ketten des monoklonalen Antikörpers B3 codiert, sowie
ii. einer veränderten Form eines bakteriellen Toxins, ausgewählt aus der Gruppe bestehend aus PE40 und PE38KDEL, wie in Beispiel II beschrieben, wobei, wenn es exprimiert wird, die genannte Fv-Region und das genannte Toxin rekombiniatorisch zusammengefügt werden, um ein einkettiges Immunotoxin zu bilden, das die Bindungsspezifität des monoklonalen Antikörpers B3 besitzt.

2. Rekombiniertes DNA-Molekül gemäß Anspruch 1, wobei das genannte Molekül ein Immunotoxin codiert, ausgewählt aus der Gruppe bestehend aus B3(Fv)-PE40 und B3(Fv)-PE38KDEL als Immunotoxin, wie in Beispiel II beschrieben.

3. Wirtszelle, die mit dem rekombinierten DNA-Molekül gemäß Anspruch 1 und/oder 2 in einer Art transformiert wurde, die das Exprimieren eines Immunotoxins zuläßt, welches durch das genannte rekombinierte DNA-Molekül codiert ist.

4. Wirtszelle gemäß Anspruch 3, wobei die Zelle eine prokaryotische Zelle, bevorzugt eine Escherichia coli-Zelle ist.

5. Rekombinatorisch hergestelltes einkettiges Immunotoxin aus:
i. der Fv-Region sowohl der leichten als auch der schweren Ketten eines Antikörpers, sowie
ii. einer veränderten Form eines bakteriellen Toxins, ausgewählt aus der Gruppe bestehend aus PE40 und PE38KDEL, wie in Beispiel II beschrieben, wobei die genannte Fv-Region und das genannte Toxin rekombinatorisch zusammengefügt werden, um ein einkettiges Immunotoxin zu bilden, das die Bindungsspezifität des monoklonalen Antikörpers B3 besitzt.

6. Immunotoxin gemäß Anspruch 5, wobei der monoklonale Antikörper B3 ist.

7. Immunotoxin gemäß Anspruch 5 und/oder Anspruch 6, wobei das genannte Immunotoxin ausgewählt wird aus der Gruppe bestehend aus B3(Fv)-PE40 und B3(Fv)-PE38KDEL, wie in Anspruch 2 definiert.

8. Medikament aus einem rekombinatorisch hergestellten einkettigen Antigenbindungsprotein, das an ein Toxin oder ein Teil davon gebunden ist, in einer Konzentration die dafür ausreichend ist, daß Tumorzellenwachstum verbinden wird, zusammen mit einem pharmazeutisch akzeptablen Trägermaterial, wobei das genannte gebundene Protein enthält:
i. eine einkettige Fv-Region des Antikörpers B3, wobei die genannte Fv-Region die V_{H}- und V_{L}- Regionen des genannten Antikörpers aufweist, sowie
ii. ein Toxinmolekül, ausgewählt aus der Gruppe bestehend aus PE40 und PE38KDEL, wie in Anspruch 1 definiert.

9. Verfahren zur Herstellung eines Immunotoxins, aufweisend die Schritte:
i. Klonen einer DNA-Sequenz, welche die Fv-Region sowohl der schweren als auch der leichten Kette des monoklonalen Antikörpers B3 codiert, in einen Vektor, wobei der genannte Vektor ein Gen enthält, das eine abgeänderte Form eines Toxins, ausgewählt aus der Gruppe bestehend aus PE40 und PE38KDEL, wie in Anspruch
1 definiert, codiert, wobei diese veränderte Form die Translokation und die enzymatische Aktivität enthält, sowie
ii. Transformieren einer Wirtszelle mit dem aus Schritt (i), erhaltenen Vektor, wobei das Exprimieren des genannten Immunotoxins zugelassen wird.

10. Rekombiniertes DNA-Molekül gemäß Anspruch 1 und/oder 2, Wirtszelle gemäß Anspruch 3, einkettiges Immunotoxin gemäß einem der Ansprüche 5 bis 7, oder Medikament gemäß Anspruch 8 zur Verwendung bei der Therapie, vorzugsweise bei der Krebsbehandlung eines Säugetiers.

11. Verwendung des rekombinierten DNA-Moleküls gemäß Anspruch 1 und/oder 2, des einkettiges Immunotoxins gemäß einem der Ansprüche 5 bis 7, oder des Medikaments gemäß Anspruch 8 zur Herstellung eines Medikaments zur Behandlung von Krebs.

## Revendications

1. Molécule d'ADN recombinant comprenant :
i. une séquence d'ADN qui code pour la région Fv des chaînes légère et lourde de l'anticorps monoclonal B3 ; et
ii. une forme modifiée d'une toxine bactérienne choisie dans l'ensemble consistant en PE40 et PE38KDEL comme décrit dans l'exemple II, dans laquelle, lorsqu'elle est exprimée, ladite région Fv et ladite toxine sont fusionnées par combinaison en formant une immunotoxine à une seule chaîne qui a la spécificité de liaison de l'anticorps monoclonal B3.

2. Molécule d'ADN recombinante de la revendication 1, ladite molécule codant pour une immunotoxine choisie dans l'ensemble consistant en B3(Fv)-PE40 et B3(Fv)-PE38KDEL qui sont les immunotoxines décrites dans l'exemple II.

3. Cellule hôte transformée par la molécule d'ADN recombinant de la revendication 1 ou 2 de manière à permettre l'expression d'une immunotoxine codée par ladite molécule d'ADN recombinant.

4. Cellule hôte selon la revendication 3, la cellule étant une cellule procaryote, éventuellement, une cellule d'*Escherichia coli*.

5. Immunotoxine à une seule chaîne produite par recombinaison comprenant :
i. la région Fv des chaînes légère et lourde d'un anticorps ; et
ii. une forme modifiée d'une toxine bactérienne choisie dans l'ensemble consistant en PE40 et PE38KDEL comme décrit dans l'exemple II, dans laquelle ladite région Fv et ladite toxine sont fusionnées par recombinaison pour former une immunotoxine à une seule chaîne qui a la spécificité de liaison de l'anticorps monoclonal B3.

6. Immunotoxine de la revendication 5, l'anticorps monoclonal étant B3.

7. Immunotoxine de la revendication 5 ou 6, ladite immunotoxine étant choisie dans l'ensemble consistant en B3(Fv)-PE40 et B3(Fv)-PE38KDEL comme défini dans la revendication 2.

8. Médicament comprenant une protéine de liaison à un antigène fusionnée à une toxine, à une seule chaîne et produite par recombinaison, ou une de ses parties, en une concentration suffisante pour inhiber la croissance de cellules tumorales, avec un véhicule pharmaceutiquement acceptable, ladite protéine fusionnée comprenant :
i. une région Fv à une seule chaîne d'anticorps B3, ladite région Fv comprenant les régions V_{H} et V_{L} dudit anticorps ; et
ii. une molécule de toxine choisie dans l'ensemble consistant en PE40 et PE38KDEL définis dans la revendication 1.

9. Méthode de production d'une immunotoxine comprenant les étapes consistant :
i. à cloner une séquence d'ADN qui code pour la région Fv de la chaîne lourde et de la chaîne légère de l'anticorps monoclonal B3 dans un vecteur, ledit vecteur comprenant un gène qui code pour une forme modifiée d'une toxine choisie dans l'ensemble consistant en PE40 et PE38KDEL définis dans la revendication 1, ladite forme modifiée conservant l'activité de translocation et l'activité enzymatique ; et
ii. à transformer une cellule hôte avec le vecteur obtenu dans l'étape (i), en permettant ainsi l'expression de ladite immunotoxine.

10. Molécule d'ADN recombinante de la revendication 1 ou 2, cellule hôte de la revendication 3, immunotoxine à une seule chaîne de l'une quelconque des revendications 5 à 7, ou médicament de la revendication 8, destiné à être utilisé en thérapie, de préférence dans le traitement du cancer chez un mammifère.

11. Utilisation de la molécule d'ADN recombinante de la revendication 1 ou 2, cellule hôte de la revendication 3, immunotoxine à une seule chaîne de l'une quelconque des revendications 5 à 7 ou médicament de la revendication 8, pour la fabrication d'un médicament destiné au traitement du cancer.
